# EUROPEAN PATENT SPECIFICATION

(11) **EP 0 800 793 B1**
(45) Date of publication and mention of the grant of the patent: **13.06.2001**
(21) Application number: 97201071.4
(22) Date of filing: 10.04.1997
(51) Int. Cl.: A61B 17/32, B24B 3/40, B23H 5/08

(54) **Process for shaping and sharpening a rotatable surgical shaver blade**
Verfahren zum Formen und Schärfen der Schneide eines rotierbaren Resezierinstruments
Procédé pour former et aiguiser le tranchant d'un appareil de résection

(30) Priority: 10.04.1996 US 636990; 30.01.1997 US 791233
(43) Date of publication of application: 15.10.1997
(73) Proprietor: LINVATEC CORPORATION, Largo, Florida 33773 (US)
(72) Inventor: Vanwyk, Robert A., No. 104-16, Largo, Florida 34647 (US); Heisler, Gary R., Holiday, Florida 34690 (US)
(74) Representative: Gambell, Derek

(56) References cited:
- US-A- 3 594 296
- US-A- 3 762 243
- US-A- 4 543 857
- US-A- 4 598 710

## Description

The invention relates to elongated, powered surgical instruments for use in endoscopic tissue resection. More particularly, the invention relates to an instrument having an elongated inner tube rotatably situated within an elongated stationary outer tube, both inner and outer tubes having, at their distal ends, cutting apertures which cooperate to resect or otherwise affect tissue during endoscopic surgical procedures. Still more particularly, the invention relates to the method of manufacturing the cutting aperture at the distal end of an elongated tubular member of a rotatable surgical instrument.

### DESCRIPTION OF THE PRIOR ART

The use of elongated surgical cutting instruments has become well accepted in performing closed surgery such as arthroscopic or, more generally, endoscopic surgery. In closed surgery, access to the surgical site is gained via one or more portals, and instruments used in the surgical procedure must be elongated to permit the distal ends of the instruments to reach the surgical site. Surgical cutting instruments for use in closed surgery -- also known as "shavers" -- conventionally have a straight, elongated outer tubular member terminating at a distal end having an opening in the end or side wall (or both) to form a cutting port or window and a straight, elongated inner tubular member concentrically disposed in the outer tubular member and having a distal end disposed adjacent the opening in the distal end of the outer tubular member. The distal end of the inner tubular member has a surface or edge for engaging tissue via the opening in the outer tubular member and in many cases (but not all) cooperates with the opening to shear, cut or trim tissue. In some cases, such as burrs, the opening in the outer tube merely allows access to the tissue and does not otherwise cooperate to resect tissue. The inner tubular member is rotatably driven about its axis from its proximal end, normally via a handpiece having a small electric motor which is controlled by finger actuated switches on the handpiece, a foot switch or switches on a console supplying power to the handpiece. The distal end of the inner tubular member can have various configurations depending upon the surgical procedure to be performed, and the opening in the distal-end of the outer tubular member has a configuration to cooperate with the particular configuration of the distal end of the inner tubular member. For example, the inner and outer tubular members can be configured to produce whisker cutting, synovial resection, arthroplasty burring or abrading, side cutting, meniscus cutting, trimming, full radius resection, end cutting and the like, and the various configurations are referred to generically as shaver blades. Cut tissue is aspirated through the hollow lumen of the inner tubular member to be collected via a vacuum tube communicating with the handpiece.

The aforementioned elongated surgical cutting instruments have also been produced in angled configurations in which the distal tips of the inner and outer members are aligned and offset or bent at either a fixed or variable angle from the proximal ends of the aligned inner and outer members. Examples of fixed and variable angle rotary surgical instruments are shown in U.S. Patents 4,646,738 (Trott) and 5,411,514 (Fucci et al.), both assigned to the assignee hereof.
In other respects the operation of fixed and variable angle shavers is largely the same as that of the straight shavers described above.

One parameter affecting the efficiency of operation of shaver blades is sharpness of the edges of the windows. Various prior art designs are known to have differing degrees of sharpness of both the inner cutting edges and the periphery of the outer window. The present invention is concerned with the design and manufacture of an outer tubular member having a sharpened window periphery.

It is known that improved resection efficiency is achieved by sharpening the cutting edges and this is true of conventional scissors as well as endoscopic shavers although the manufacture of the latter is considerably more difficult. The smaller the included angle of the cutting edge, the sharper the edge. Clearly, below a certain limit the edge becomes too delicate to be practical. When applied to the tubular members of cylindrical, rotating shavers, the cutting edges on the inner member and the periphery of the outer window are the cooperating edges which should have the smallest included angles in order to produce sharp edges. However, this must be balanced with cost and speed of manufacture. In prior art designs, the outer window is sometimes formed by simply grinding or milling an opening at the distal tip of the outer tube, the opening lying in a plane angled relative to the tube axis. Thus, the outer window faces toward the end of the tube as well as toward the side. It will be understood that this process produces a generally elliptical window periphery which has a lower included angle at its proximal end and a larger included angle at its distal end. A land surface surrounds the periphery and is angled (in the cutting plane) such that the inner rim of the land defines the sharpened cutting edge of the outer member. The formation of this type of outer window could be achieved by a variety of two-dimensional through-cutting processes such as wire EDM (electrical discharge machining), ram EDM, conventional or electrochemical grinding or milling. For certain purposes, this type of cutting window may be sufficient, however, it is known that subjecting the land surface to additional processing can produce a sometimes more preferable "three-dimensional" window shape and sharper edge. The term "three-dimensional" is used to distinguish the opening from one formed by a simple planar cut through the tip of a tube: the periphery of the latter lies only in a two-dimensional plane while the periphery of the former also extends above and below this plane. However, the additional steps required to produce such sharpness entail the use of either a tool having a complex contour or a machining process capable of complex contouring motions (e.g., a computer numerical control (CNC) machine). Additionally, practical manufacture of these devices would be hampered because simultaneous production of several blades is more difficult with such processes, if at all possible.

In U.S. Patent 4,543,857 (Kleinberg et al.) corresponding to the preambles of the independent claims there is disclosed a surgical instrument in which a pair of co-axial tubular members include cooperating oval openings the formation of which are developed by an apparatus comprising a fixture for supporting the inner or blade member in a spindle having a follower, and a cam assembly and registry means for mounting to the fixture. The cam assembly is mounted to the fixture and through cooperative action with the follower directs movement of the blade member and spindle during grinding formation of a blades's thickness including a positive cutting angle across its wall. The registry means comprises a body mountable to the cam assembly and having a pin which properly seats an initially formed opening in the blade member exteriorly of the cam assembly for subsequent grinding thereon. A second apparatus provides for the initial formation of both openings after which the first apparatus operates to finish the blade member's body formation for its opening.

In U.S. Patent 4, 598, 710 (Kleinberg et al.) there is described a surgical instrument comprising a pair of coaxially assembled tubes having their distal walls in bearing relationship and with registrable openings extending through such distal end and annular walls correspondingly joined to their respective distal walls. The inner tube reversibly rotates within its matched outer tube as action by cutting edges formed about such openings severs tissue drawn into the inner tube by suction. Each distal end wall includes a pair of circular cutting edges, those in the inner tube having a straight cut across its thickness while those in the outer tube have a full radiused cut across its thickness. Full radius cuts across the thickness of the annular walls of the inner and outer tubes are provided for arcuately-formed and parallel cutting edges forming the respective openings in these tubes in their annular walls.

It is accordingly an object of this invention to produce an outer member of a shaver blade assembly in which the window of the outer member has a sharpened periphery, formed by a peripheral land surface angled to the window rim with a simple process minimizing the use of complex tools and the number of required processing steps.

With the present invention a three-dimensional window is shaped and sharpened in a tubular member of a rotatable surgical instrument with a single step process.

### SUMMARY OF THE INVENTION

These and other objects of this invention are achieved by the preferred embodiments disclosed herein which are methods of forming an opening at one end of an elongated tubular surgical shaver blade having a distal end and a proximal end. In one embodiment, the method comprises the steps of:
providing an apparatus for performing an electro-chemical grinding process on a portion of the elongated tubular surgical shaver blade, the apparatus having a rotatable abrasive wheel with a perimetral surface which has a circumferential groove formed therein, said groove having a predetermined arcuate profile in a radial plane of said wheel;
orienting the distal end of the elongated tubular surgical shaver blade in a predetermined orientation relative to said wheel;
activating said apparatus to perform an electro-chemical grinding process; and
moving the distal end of the elongated tubular shaver blade relative to said wheel during the performance of said electrochemical grinding process to perform the electrochemical grinding process on the distal end of the elongated tubular surgical shaver blade to thereby form an opening therein, where the opening has a peripheral land angled around the entire periphery of the opening and where the periphery has a sharp edge without necessity for a subsequent sharpening operation.

In another aspect the invention lies in a method comprising the steps of:
providing a hollow tubular member having an axis, a distal end and a proximal end;
providing a grinding wheel having an axis and a predetermined arcuate groove in its perimetral surface, said groove for creating in said tubular member an arcuate surface having at least one predetermined radius of curvature;
orienting said axis of said predetermined portion of said tubular member at a predetermined angle relative to a line tangent to said wheel;
rotating said grinding wheel about its axis;
advancing said predetermined portion of said tubular member along a path which intersects with a selected portion of said grinding wheel whereby such motion will result in an opening being shaped in said predetermined portion of said tubular member, where the opening has a peripheral land angled around the entire periphery of the opening and where the periphery has a sharp edge without necessity for a subsequent sharpening operation.

A further aspect of the invention relates to a method of shaping an opening in a tubular member of a rotary shaver blade comprising the steps of:
providing a cylindrical forming apparatus having a circumferential surface for shaping in said tubular member an opening, said circumferential surface situated at a predetermined radius from a first axis and being movable about said first axis;
fixedly mounting a hollow tubular member with its axis parallel to said first axis;
rotating said axis of said tubular member about a second axis parallel to said first axis, the distance between said first and second axes being greater than said predetermined radius and such that during rotation of said axis of said tubular member about said second axis a predetermined portion of said tubular member will intersect a predetermined portion of said circumfer-ential surface such that an opening will be shaped, where the opening has a peripheral land angled around the entire periphery of the opening and where the periphery has a sharp edge without necessity for a subsequent sharpening operation.

A further aspect of the invention relates to a method of forming a window in a rotary shaver blade comprising the steps of:
providing a hollow tubular member having a distal end;
providing an elongated arcuate surface forming means comprising a first elongated arcuate concave surface which is linear in the direction of elongation and which has a predetermined transverse curvilinear shape for, upon operation thereof, creating in said hollow tubular member at least one second arcuate convex surface complementary to a predetermined portion of said first arcuate surface;
orienting said first arcuate concave surface at a predetermined orientation relative to and facing said hollow tubular member;
advancing said first arcuate concave surface toward said end and operating said first arcuate concave surface to cause it to remove a predetermined portion of said hollow tubular member to create in said hollow tubular member a window defined by the intersection of said hollow tubular member and said first arcuate surface, where the window has a peripheral land angled around the entire periphery of the window and where the periphery has a sharp edge without necessity for a subsequent sharpening operation.

### BRIEF DESCRIPTION OF THE DRAWINGS

Figure 1 is a side elevational view of the distal tip of a prior art outer tube having a curvilinear cutting window.

Figure 2 is an end view of Figure 1.

Figure 3 is a plan view of Figure 1.

Figure 4 is a side elevational view of a distal tip of an outer tube formed in accordance with the principles of this invention.

Figure 5 is an end view of Figure 4.

Figure 6 is a plan view of Figure 4.

Figure 7 is a front perspective view of the outer member shown in Figures 4-6 with a chosen inner member.

Figure 8 is a diagrammatic plan view of Figure 7.

Figure 9 is a sectional view of Figure 8 taken along the line 9-9.

Figure 10 is a sectional view of Figure 8 taken along the line 10-10.

Figure 11 is a sectional view of Figure 8 taken along the line 11-11.

Figure 12 is a sectional view of Figure 8 taken along the line 12-12.

Figure 13 is a diagrammatic elevational view in cross-section of an apparatus and method step incorporating the principles of this invention.

Figure 14 is a sectional view of Figure 13 taken along the line 14-14.

Figure 15 is a view of the method and apparatus of Figure 13 in a different stage of the process of this invention.

Figure 16 is a sectional view of Figure 15 taken along the line 16-16.

Figure 17 is a view of Figure 15 taken at a different stage in the process of the invention.

Figure 18 is an end view of Figure 4 taken along the line 18-18 showing the profile of the tip viewed along a line angled relative to its axis.

Figure 19 is a diagrammatic front elevational view of an electrochemical grinding apparatus used in a preferred embodiment of the invention.

Figure 20 is a view of Figure 19 taken along the line 20-20.

Figure 21 is an enlarged view of a portion of Figure 20.

Figure 22 is a side elevational view of the distal end of a prior art toothed outer member.

Figure 23 is a cross-sectional view of Figure 22 taken along the line 23-23.

Figure 24 is a cross-sectional view of Figure 22 taken along the line 24-24.

Figure 25 is a side elevational view of the distal end of an outer tubular member constructed in accordance with the principles of this invention.

Figure 26 is a cross-sectional view of Figure 25 taken along the line 26-26.

Figure 27 is a cross-sectional view of Figure 25 taken along the line 27-27.

Figure 28 is a side elevational view of an apparatus used in the method of producing a toothed outer such as that shown in Figure 25.

Figure 29 is a front elevational view of Figure 28.

Figure 30 is an enlarged view of a portion of Figure 29.

Figure 31 is a side elevational view of the components of Figure 28 showing a portion of the method of producing the toothed outer member of Figure 25.

Figure 32 is a view of Figure 31 at a different portion of the method.

Figure 33 is an exploded view of Figure 30 showing in phantom the path followed by various portions of the outer tubular member during the manufacturing process.

Figure 34a through 34g show various positions of the components of the invention during various portions of the method used to form the toothed outer tubular member.

Figure 35a and 35b show cross-sectional views of a prior art rotatable surgical shaver having an outer tubular member such as that shown in Figures 22-24 and an inner tubular member having a cutting window formed by a straight cut.

Figure 36a and 36b are cross-sectional views, of a rotatable surgical shaver utilizing an outer tubular member constructed with the principles of this invention and a prior art inner tubular member having a cutting window with a straight cut.

Figure 37 is a cross-sectional view of an inner tubular member during a process of forming sharpened edges around an opening of the member.

Figure 38 is a side elevational view of Figure 37.

### DESCRIPTION OF THE PREFERRED EMBODIMENT

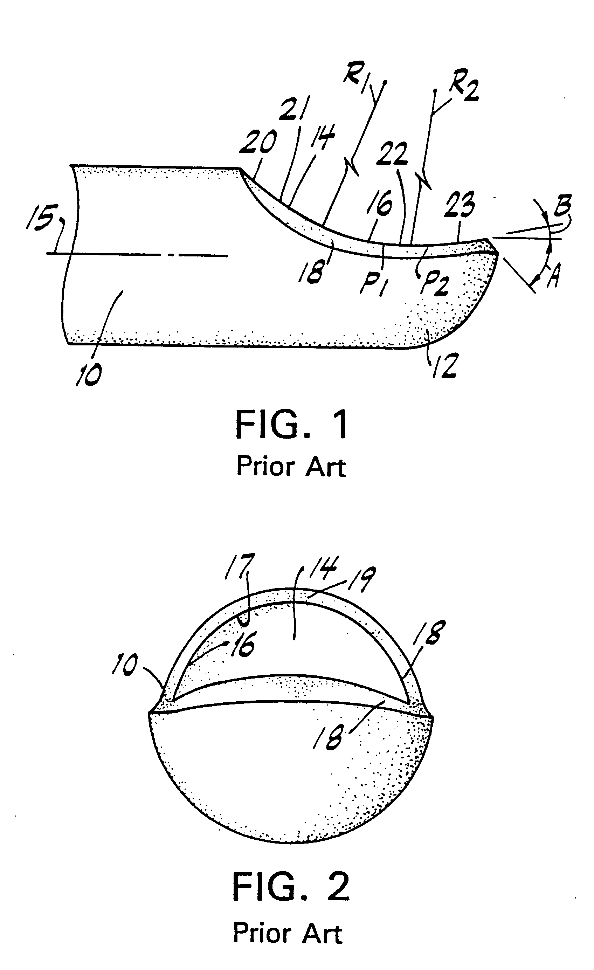

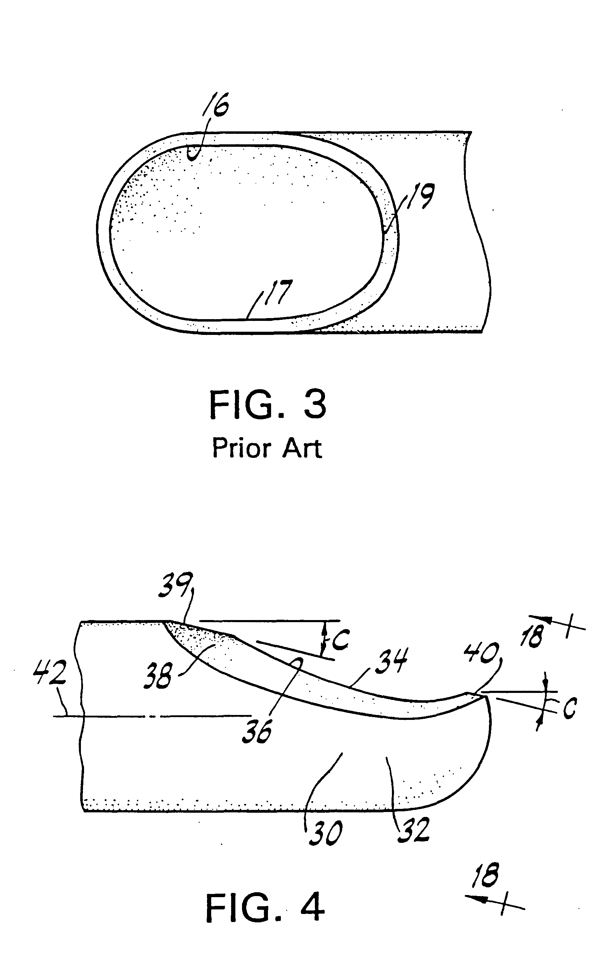

Figures 1 and 2 show a distal tip of a prior art outer tubular member 10 of a rotary shaver blade assembly (not to scale). The proximal ends of the tubular member are conventional and well understood by those skilled in the art. Outer tubular member 10 has a distal tip 12 with a curvilinear cutting window 14 which faces to the side, i.e. laterally, perpendicular to axis 15, and away from the end of the blade, i.e. longitudinally in a direction parallel to axis 15. The terms "laterally" and "longitudinally" mean that the interior of the outer tubular member is visible when viewed from these vantage points. The window has a peripheral rim 16 which is formed by the intersection of the window with the interior surface 17 of the outer tube and is surrounded by a land 18 which is angled at its distal end at an angle A relative to axis 15 and the distal tip of window periphery 16. Angle A appears to be formed by a complex grinding or shaping process and appears to be uniform along the extent of the land surface. Thus, land 18 lies at angle A relative to the rim at each point along periphery 16. It is noted that proximal end 20 of window 14 is not provided with an angled land at all, thus resulting in portion 19 which is simply the end facing surface of the outer tube wall which is exposed by the cut forming window 14. The curvilinear profile of window 14 is best seen in Figure 1 as comprising three sections: a first section 21 extending from the proximal end 20 of the window to a first predetermined point P1, and having a radius of curvature R1; a second section extending from point P1 to a predetermined point P2 adjacent the distal end of the window, this second section 22 having whatever blended radius of curvature R2 which is necessary to smoothly join the radiused section 21 to a third section 23 which is a linear portion extending at some angle B relative to axis 15. The window thus formed is best seen in Figure 3 as having a relatively rectangular shape with rounded proximal and distal ends and generally parallel sides. Window 14 is formed by a process having at least two steps: a first step requiring passing a cutting tool through the distal tip of tube 10 along the curvilinear profile shown in Figure 1 and a second step requiring the formation of land 18.

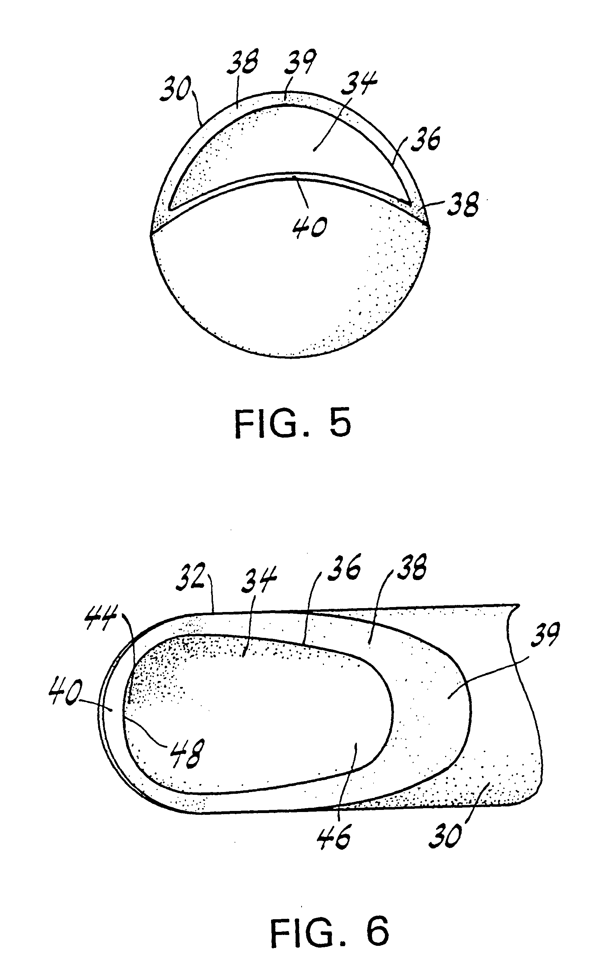

The subject invention relates to an improved outer tube 30 and a process for forming the tube as shown in the remaining drawings. Referring to Figures 4 and 5, outer tube 30 has a distal end 32 provided with a curvilinear window 34 having a peripheral rim 36. The proximal end of tube 30 is conventional and forms no part of this invention. Window 34 is surrounded by a peripheral land 38 angled at its distal and proximal ends 39 and 40, respectively, at an angle C relative to the axis 42 of the outer tube. It will be noted that land 38 is, unlike the prior art embodiment discussed above, angled around the entire periphery of the window although points intermediate the distal and proximal ends 39 and 40 may be at angles other than angle C as will be understood below. As best seen in Figure 6, window 34 is less rectangular than window 14 of the prior art embodiment and has more of a pear-shaped or generally elliptical outline with a large rounded distal end 44, a smaller rounded proximal end 46 and non-parallel sides. It should also be noted that the invention facilitates creation of a large window while maintaining a distal bearing tip 48 on axis 42. Tip 48 may act as a bearing surface against the exterior surface of the inner member.

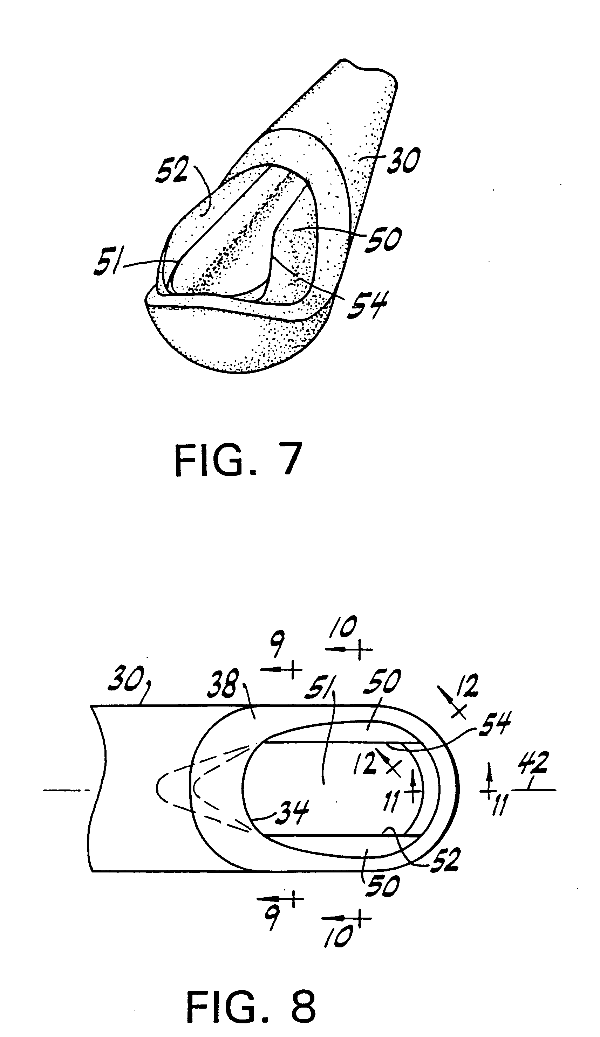

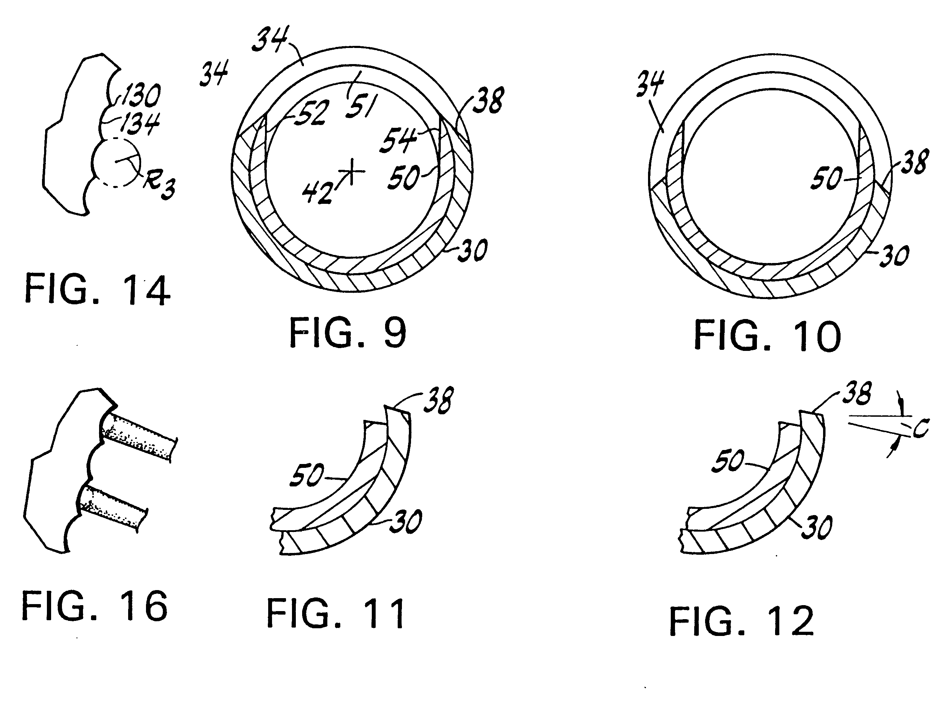

Figure 7 is a front perspective view of the distal tip of outer tubular member 30 assembled with an inner member 50 having a cutting window 51 although it will be understood that a variety of inner cutting window profiles could be used. To facilitate the explanation of the invention, Figure 7 is presented in diagrammatic plan view in Figure 8 showing the various points at which cross-sectional views shown in Figures 9 through 12 are taken.

The particular form of cutting edge provided on inner member 50 has a pair of inwardly facing cutting surfaces 52 and 54 created on either side of inner window 51. Since each cutting surface 52 and 54 is close to if not exactly tangential with the interior surface of the inner tube (best seen in Figure 10) and extends proximally from the distal tip of the inner tube, the inner window has a generally rectangular opening when viewed from the front. The inner window is formed, for example, by a wire EDM process in which the wire is longitudinally extending at a predetermined angle to the axis of the inner member and follows a generally rectangular path (viewed from the end of the tube). This path produces a tangential cutting surface at only certain points (i.e. at Figure 10 but not at Figure 9). The relationship of the cutting surfaces 52 and 54 relative to adjacent portions of outer window 34 is better seen by reference to Figures 9 through 12 which show sectional views through varying portions of Figure 8. It will be noted that the angle of land 38 relative to a horizontal plane through axis 42 varies depending upon the longitudinal placement of the point on the land at which the angle is measured. Thus, as shown in Figure 9, the edge of outer window 34 is fairly sharp because the wall of the outer tube is cut at a relatively low included angle compared to the angle of land 38 at other points, such as the point shown in Figure 10. Similarly, the angle of land 38 at the point represented by Figure 11 is different still and the angle shown in Figure 12 is the angle C referred to above. It will be further noted that the relatively low included angle forming the cutting edge of the inner cutting window, i.e. the intersection of surfaces 52 and 54 with the outside surface of inner member 50 results in a sharp edge in the areas of Figures 9 and 10. The curvilinear contour of outer window 34 in combination with the sharpness of its edges along rim 36 and the edges of the inner window produces efficient tissue resection.

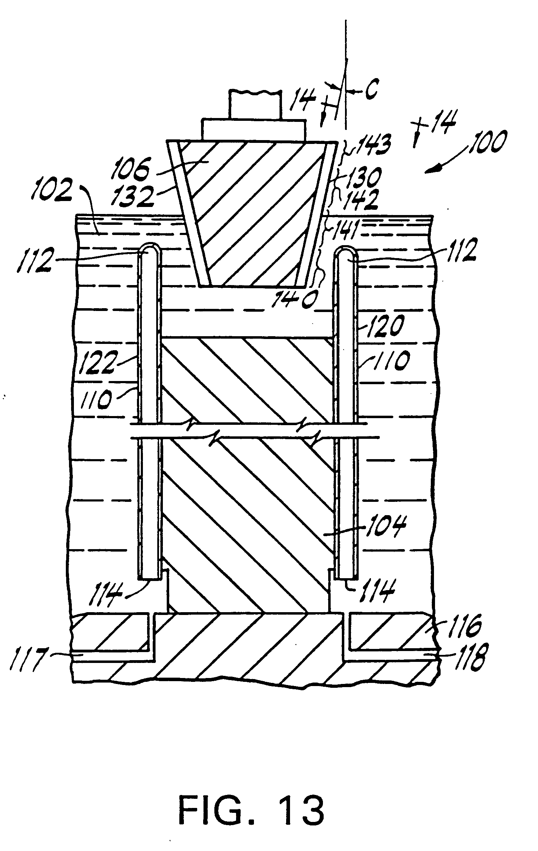

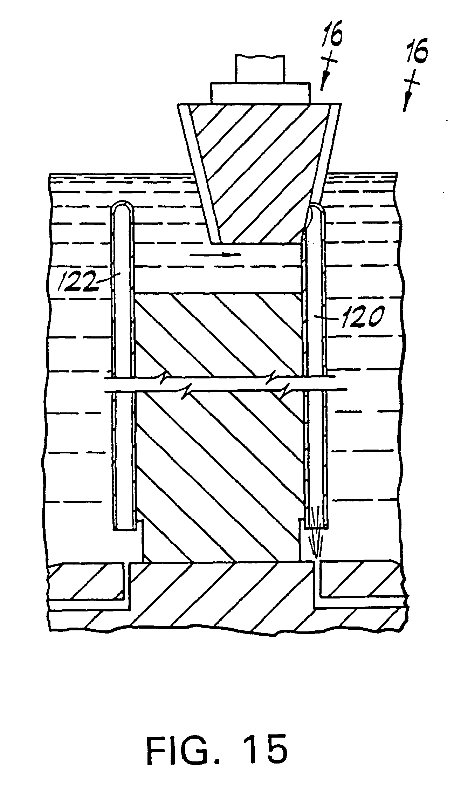

One process of forming the curvilinear contour of window 34 at distal tip 32 is best understood by reference to Figures 13 through 18. The process utilizes a contouring apparatus such as an electrical forming device capable of creating an arcuate surface in another body. The embodiment disclosed utilizes a plunge-type conventional computer numerically controlled (CNC) EDM device 100 incorporating a basin (not shown) for holding dielectric fluid 102, a work piece holder 104 and forming electrode tool 106 mounted to the EDM upper platen. A plurality of hollow outer tubes 110 are attached in a vertical orientation to opposite sides of holder 104, each outer tube having a closed top end 112 and an open bottom end 114. Holder 104 is attached to a base 116 mounted to the EDM lower platen, the base being provided with a plurality of fluid channels 117 and 118 aligned with the open ends of associated tubes 110. While only two tubes 110 are shown in Figure 13, it will be understood that additional tubes are arranged on holder 104 in two parallel rows 120 and 122 which may extend perpendicularly to the plane of the paper. Similarly, tool 106 is an electrode having a trapezoidal cross-section, best seen in Figure 13, extending perpendicularly to the plane of the paper and above holder 104. The angled sides 130 and 132 of tool 106 are each provided with a plurality of parallel channels 134 having arcuate profiles oriented at angle C relative to the axes of the tubes in rows 120 and 122 , each having a radius of curvature R3, best seen in Figure 14, which ultimately interacts with outer tubes 110 to create the unique profile of window 34. It will be understood that the channels could have other than circular profiles.

As shown in Figure 15, electrode tool 106 is first moved down to a selected level between the parallel rows 120 and 122 of outer tubes 110 and then laterally toward one of the rows, the rate being determined by the machine control parameters in order to have the distal tip of outer tubes 110 contact a selected channel 134. While a plurality of channels are provided on each surface 130 and 132, not all portions of all channels need to be used at any one time and the tool may be shifted around by proper programming of the CNC machine to utilize selected portions of the channels and selected channels as will be understood below. For example, any given channel 134 may be thought of as having a plurality of adjacent sections 140, 141, 142 and 143. The vertical positioning of electrode 106 could be, for example, set to have lower-most section 140 be the active section. As the electrode continues to move laterally a predetermined distance to a selected point, the EDM process forms an opening in each of the outer tubes while dielectric fluid is flushed through channels 118 and out the opening created at the distal tip. During machining, electrical discharge parameters are modified under program control so as to minimize cycle time while maintaining acceptable surface finish and edge sharpness on the finished product. The pressurized dielectric fluid introduced by the timed flushing port reduces the machining time by removing swarf from the machining zone. The opening is defined by the intersection of the cylindrical tube wall at the rounded, closed end of the tubes with the arcuate profile of channels 134.

When the tool has been moved laterally a programmed distance to form the desired window 34, the direction of the tool is reversed to approach row 122 and the channels along surface 132 are brought into engagement with the outer tubes in row 122 in order to produce the chosen profiles in those tubes.

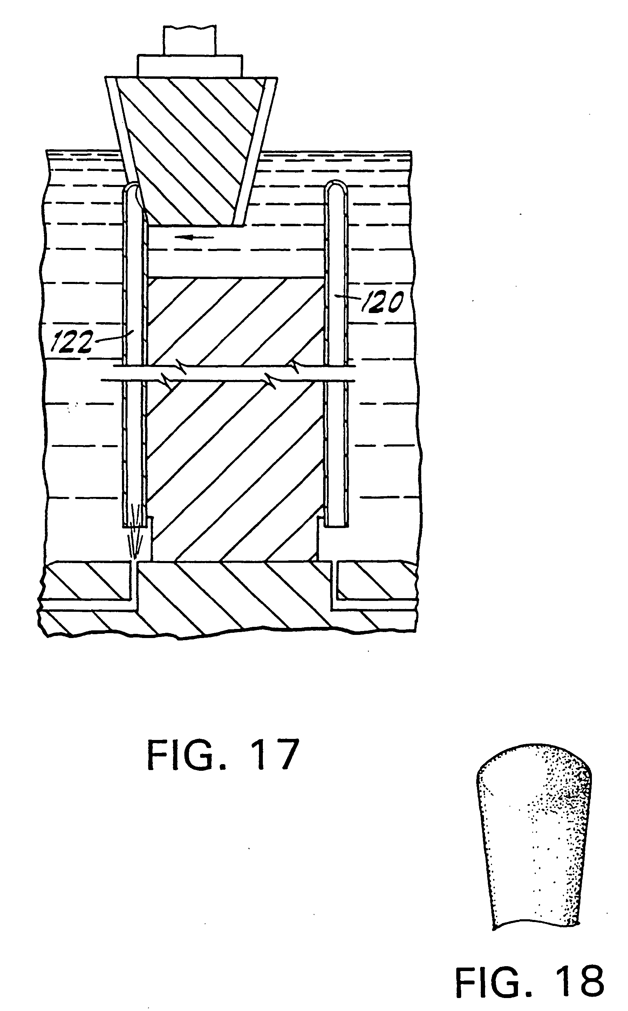

It will be appreciated by those skilled in the art that while tool 106 has a relatively simple shape and moves in a relatively simple pattern, the intersection of the tool with the outer tube produces a resultant complex profile. Rather than being defined by a complex shape requiring several processing steps for its formation, window 34 has a simple arcuate profile when viewed in Figure 16, or better still, when viewed along a line at an angle C to the tube axis as best seen in Figure 18. The intersection of the arcuate channel profile with the cylindrical tube results in cutting edges with low included angles thereby making a subsequent sharpening operation unnecessary. The shape of the surface of any given land 38 surrounding rim 36 and the shape of the associated actual window 34 may be considered as being defined by the locus of all points lying at the intersection of an elongated, transversely arcuate surface with the cylindrical body of the tube oriented at a predetermined angle relative to the transversely arcuate surface. Put another way, the definition of the perimeter of window 34 may be thought of as the intersection of a cylindrical body with an imaginary surface having a predetermined, symmetrical, concave arcuate profile. In practice this imaginary surface is defined by the concave surface of channel 134 and is considered endless because the surface extends entirely through the cylindrical body enough to form a window therein. In the preferred embodiment, however, the activatable sections (140, etc.) of the surface of channel 134 lie on this imaginary surface and need only subtend a lateral or transverse distance approximately greater than or equal to one-half the outside diameter of the cylindrical body in order to form land 38. If the imaginary surface is linearly extended in a direction which is aligned with the axis of the tube, and if the arcuate profile transverse to the axis is a simple concave radius, the resultant locus of points defines convex land 38 (complementary to the concave radius). If some other orientation or profile is used, the locus of points will produce a different shape.

The transverse curvature of window 34 when viewed as shown in Figure 16 is the same as that shown in Figure 18. While the arcuate profiles of channels 134 are at a constant radius of curvature along the length of the channels, it will be understood that the radius of curvature may vary along the channel length and/or the arcuate surface may have a contour other than circular extending along the axis line.

Because the shape is two-dimensional when viewed axially to the cylindrical surfaces 130 and 132 of the tool, complex shaped tooling or simultaneous programmed machine motions are not required. The shape of the tool may be machined by using CNC wire EDM, or produced by conventional or electrochemical grinding using a wheel onto which the proper radius has been dressed. Similarly, plunge type EDM or ECM may be used with the part contour being produced by contours on the tooling. Tooling cost is low due to the simple profile and, because complex machine motions are not required, simultaneous multiple part machining is readily implemented.

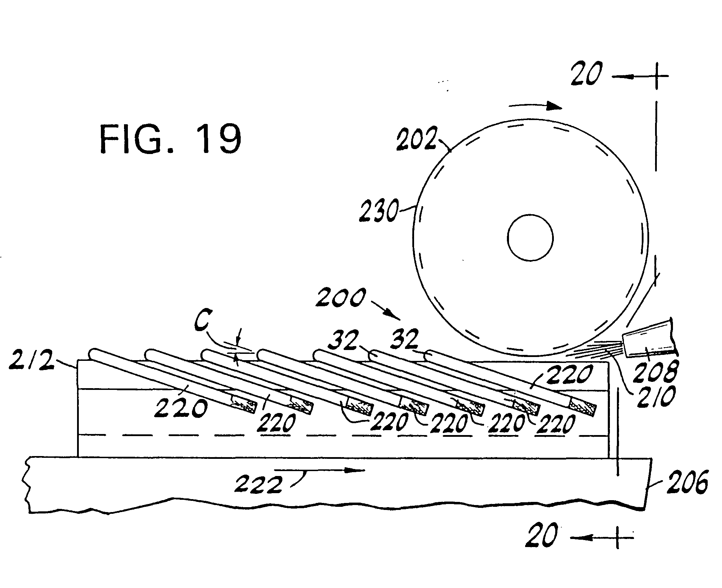

A preferred process of forming the curvilinear contour of window 34 at distal tip 32 is best understood by reference to Figures 19-21. This process utilizes a cylindrical forming apparatus which, in the preferred embodiment, is a conventional electrochemical grinding machine (ECG) which has had the perimetral (circumferential) surface of its abrasive grinding wheel shaped to ultimately form the contour of window 34. It will be understood that the processes disclosed herein could be used to form an opening in a previously closed distal end of a tubular member, or could be used to shape or sharpen an opening previously formed therein.

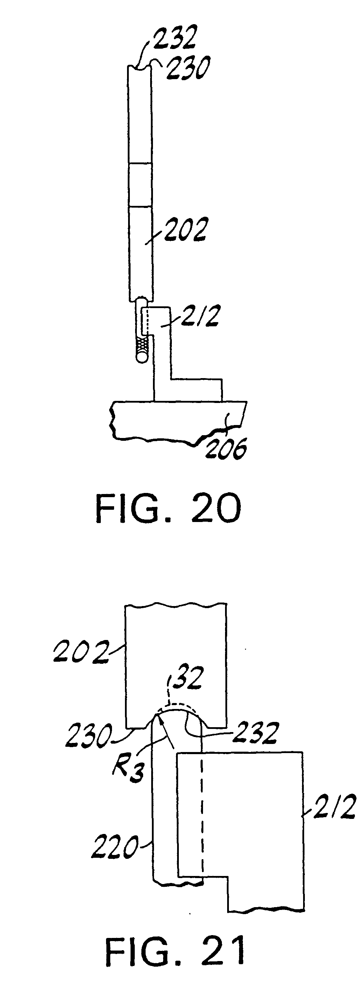

Electrochemical grinding machine 200 comprises a grinding wheel 202, a base (not shown) for supporting a table 206 movable relative to the base and wheel under programmable control. A nozzle 208 directs electrolyte 210 at the work location adjacent wheel 202 in a conventional manner. The operation of the machine components, processes, table motion, speed, etc. is controlled in a conventional manner. Table 206 receives a jig 212 to hold a plurality of tubular members 220 in an inclined orientation relative to a line tangent to wheel 202. The tubular members 220 are electrically conductive and serve as one electrode within the apparatus (the wheel acting as the other electrode). Each member 220 has a closed distal tip 32 into which a window 34 (as shown in Figures 4-6) will be formed as explained herein. The tubular members 220 are inclined at an angle C which is the same as the angle between the tubular axis and the face of the channels of tool 106 as shown in Figure 13. As table 206 moves in direction 222 the distal tips of successive tubular members 220 will move tangentially past wheel 202. Perimetral surface 230 of wheel 202 is provided with a groove 232 having the selected profile which in this embodiment is a simple radius of curvature R3, the same radius as that of channel 134 shown in Figure 14. Groove 232 is best seen in Figure 21 which shows a view of the wheel groove within essentially a radial plane of the wheel. Moving table 206 along a linear path of motion in the plane of wheel 202 to tangentially intersect distal tips 32 of tubular members 220 with the groove at the wheel periphery during activation of the electrochemical process will produce in each tip a curvilinear window 34 as shown in Figures 4-6 and 18 without any additional shaping or sharpening process. While jig 212 and table 206 are shown in the drawings to fixedly hold each tube in a fixed orientation relative to wheel 202, as will be explained below the tubes could be supported on jig 212 in a movable manner and the table could move along a complex path of motion (other than simply in one linear direction). The movement of the tubes relative to the jig or the movement of the table relative to the base could be continuous or indexed (to discrete positions) under programmable control. While the apparatus of Figures 19-21 utilizes electrochemical grinding, similar wheel shapes and motions may be applied to conventional grinding or other processes.

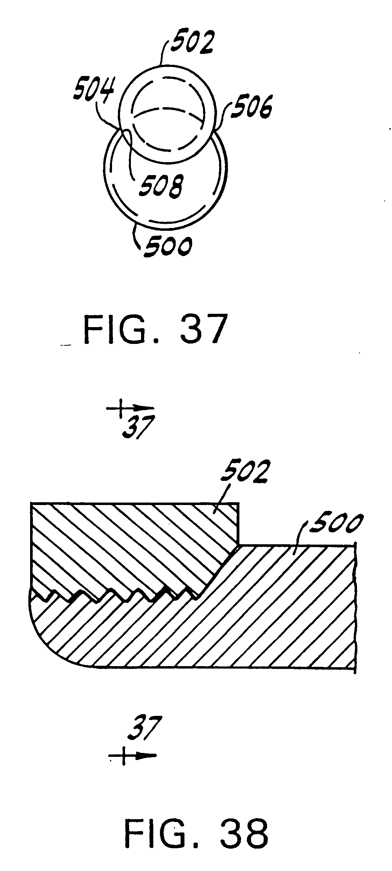

While the device and process disclosed have been described in terms of an outer, stationary tubular member, it will be understood by those skilled in the art that the cutting edge of an inner, rotating tubular member may also be formed in accordance with the principles of the invention disclosed herein. Appropriate changes may be necessary in the shapes and sizes of the electrode tool 106 or the wheel 202, supporting jigs and paths of travel, depending upon the desired shape of the inner cutting edge. One such device and process is disclosed below in Figures 37 and 38.

Furthermore, the window shape of the preferred embodiment of the outer tube disclosed herein is such that a linear tangential motion of the inclined tubular members 220 relative to wheel 202 is the desired motion to produce the desired shape. Variations in window shape or cutting edges in either the inner or outer members may require other orientations of the tubular members or other than linear motion of table 206.

Also, as briefly mentioned above, the path of motion of the tubes relative to the wheel may change depending upon the desired shape to be formed in a tubular member. For example, the tube may be moved in varying directions other than linear and/or it may be rotated about its axis as it moves past the wheel, moved up and down relative to the wheel on any given pass to change the depth of the cut in the tube, etc. Additionally, the machine could have different ECG stages, each with a different wheel so that a complex table path of motion would pass differently shaped wheels to produce complex cuts and different shapes. Another example may be a tube formed by a plurality of linear passes. That is, the tube could be moved linearly relative to a wheel as described above, rotated about its axis, moved linearly again, rotated again, moved again, etc. The wheel in such case could even have a flat peripheral surface to produce an opening approximating window 34. With appropriate indexing and motion of the tubular member in all dimensions relative to the jig (e.g. linearly along its axis, rotating about its axis and about another axis transverse to its axis) and with appropriate indexing and motion of the table relative to the wheel (or relative to different wheels if additional stages are used), it would be possible to produce cuts of almost any imaginable shape, depth, angle and length such that this method would even make it possible to produce cutting teeth in the tubular member. This latter method could be analogous to creating a complex shape by a CNC (computer numerical control) milling machine operating with several degrees of freedom and would result in a toothed opening in either an inner or outer tubular member of a rotating surgical shaver.

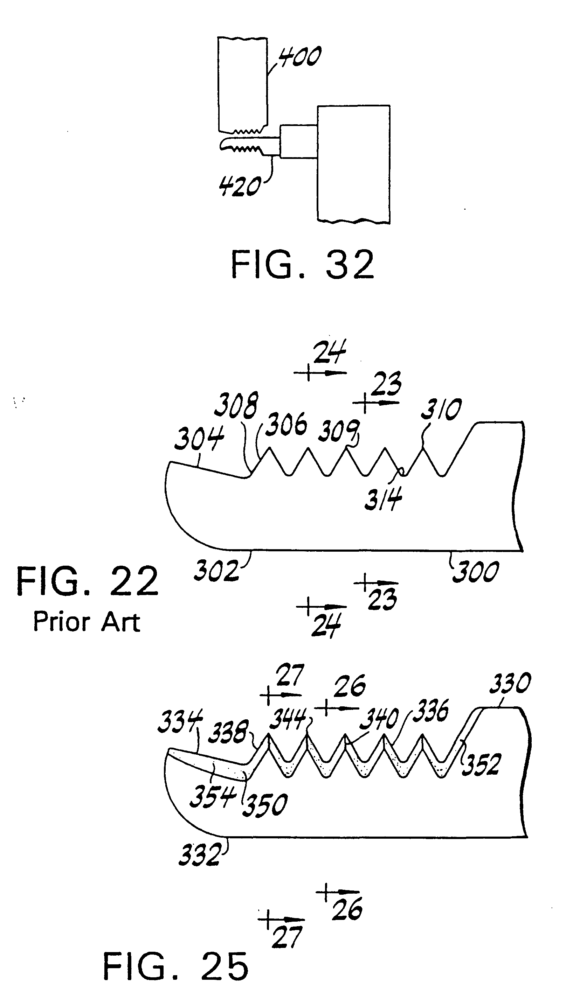

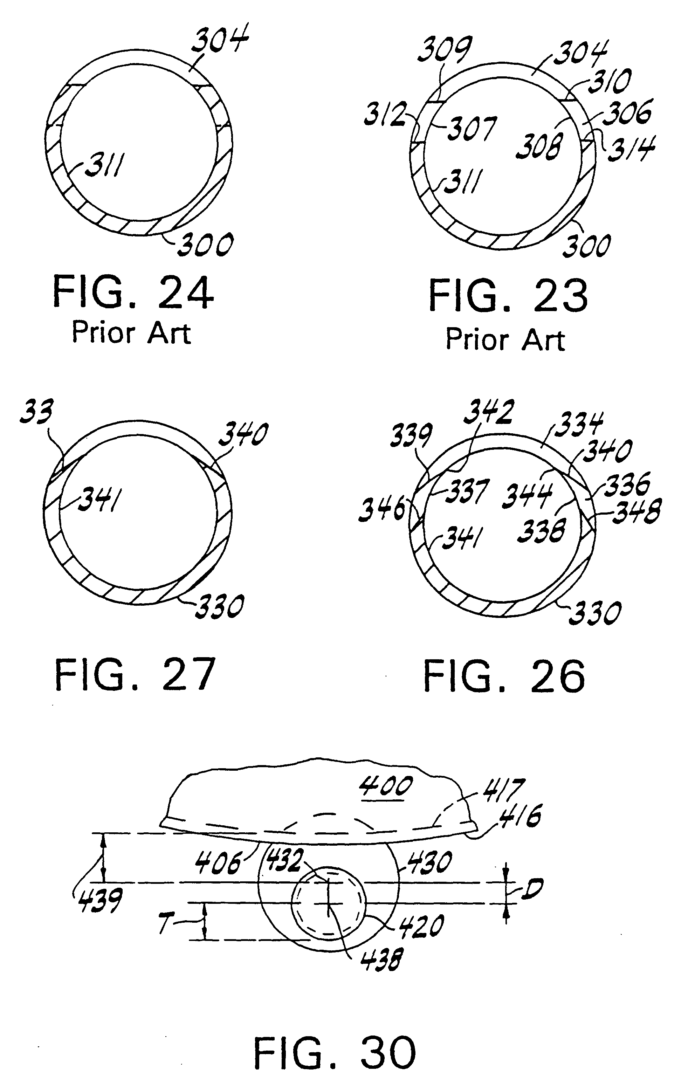

One method of forming the cutting teeth in a tubular member is shown in Figures 22 through 36. As shown in Figures 22-24 a prior art outer tubular member 300 has a distal tip 302 comprising a cutting window 304 bounded a plurality of teeth 306 in two, parallel and longitudinally extending rows 307 and 308. The cutting window and teeth of such an outer tubular member 300 may be formed, for example, by a conventional EDM process in which the cut is made transversely through the distal tip of the tubular member so that the top edges 309, 310 of teeth in opposing rows 307, 308 are coplanar at corresponding transverse points and the troughs 312 and 314 between adjacent teeth in opposing rows are also coplanar. The included angle of each tooth is bounded by the horizontal surface 309 (or 310, depending on the row) and the inner surface 311 of the tube.

As shown in Figures 25 through 27 outer tubular member 330 constructed in accordance with the principles of this invention has a toothed profile considerably sharper than that of prior art tubular member 300. Outer tubular member 330 has a distal tip 332 in which a cutting window 334 is formed which is bounded by a plurality of teeth 336 in two, parallel and longitudinally extending rows 337 and 338. As with the prior art device, the teeth 336 in one row are transversely coplanar with the corresponding teeth in the opposite row. The top edges 339 and 340 of each tooth 336 are curved to produce points 342 and 344. Troughs 346 and 348 between adjacent teeth are also curved but at a different profile as will be understood below. The included angle between edge 339 and the inner surface 341 is smaller than the corresponding angle of the prior art device 300.

Cutting window 334 has a land 350 entirely surrounding the window, the land being angled relative to the window periphery so that there is a sharp edge on the radially innermost side adjacent to the opening. The included angle between the land surface and the inner surface 341 varies depending upon the longitudinal position of the point where the angle is measured. For example, the included angle at edge 339 is smaller than that at trough 346. Land 350 comprises a proximal land section 352 and a distal land section 354, the shapes of which are shown diagrammatically. It will be understood that, while an inclined land surface will surround window 334, the actual shape of this land may vary from that shown in Figure 25 depending upon the actual tool used.

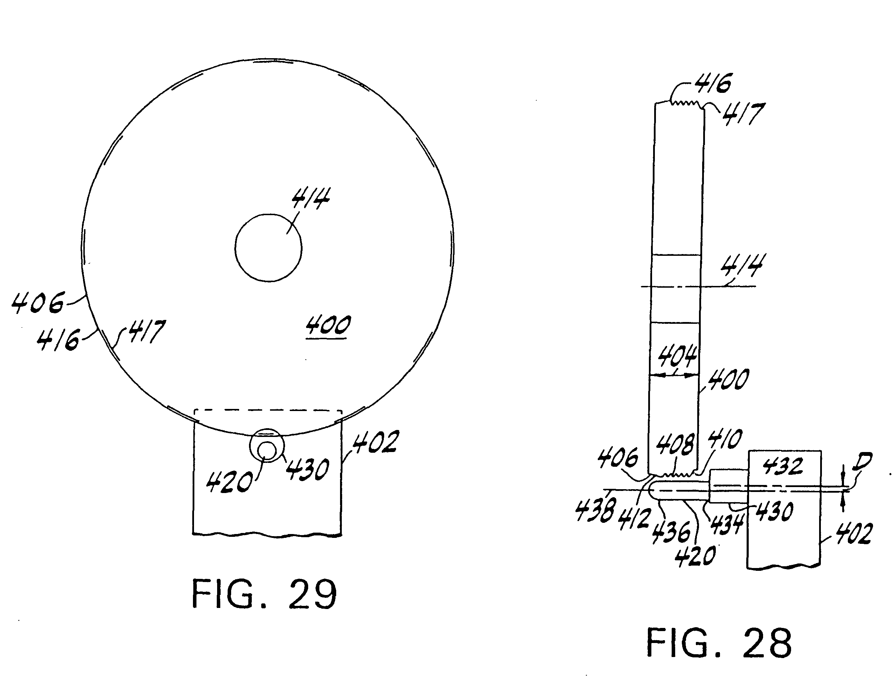

Figures 28-33 and 36 show an apparatus and method for producing the toothed outer tubular member 330. Wheel 400 and jig 402 are part of electrochemical grinding machine similar to that shown in Figure 19. The remaining components (table, electrolyte nozzle, etc.) are omitted for clarity. Wheel 400 has a predeter-mined thickness 404 and a predetermined perimetral surface 406 which is dressed with a plurality of notches 408 and proximal and distal angled surfaces 410 and 412, respectively, to form the various teeth 336 and proximal and distal land sections 352 and 354 as will be understood below. The shapes of surfaces 410 and 412 in combination with the motions of various components will affect the shape of proximal and distal land sections 352 and 354. The various cuts in the perimetral surface 406 have a radially outermost portion (extent) 416 and a radially innermost portion (extent) 417. The shape of the perimetral surface may be changed to produce a variety of cuts in a workpiece 420 as will be understood below.

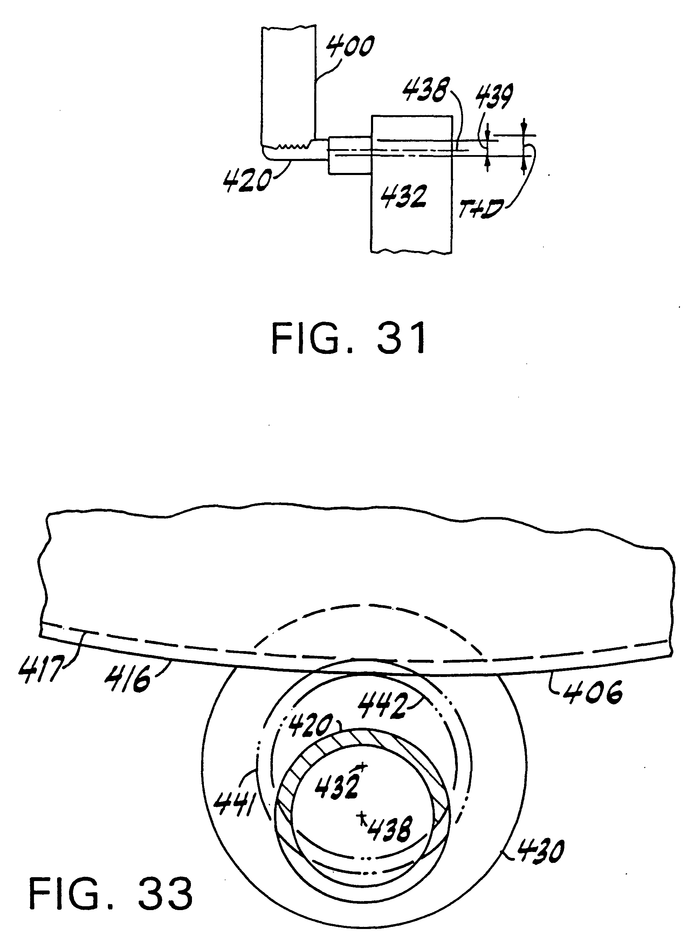

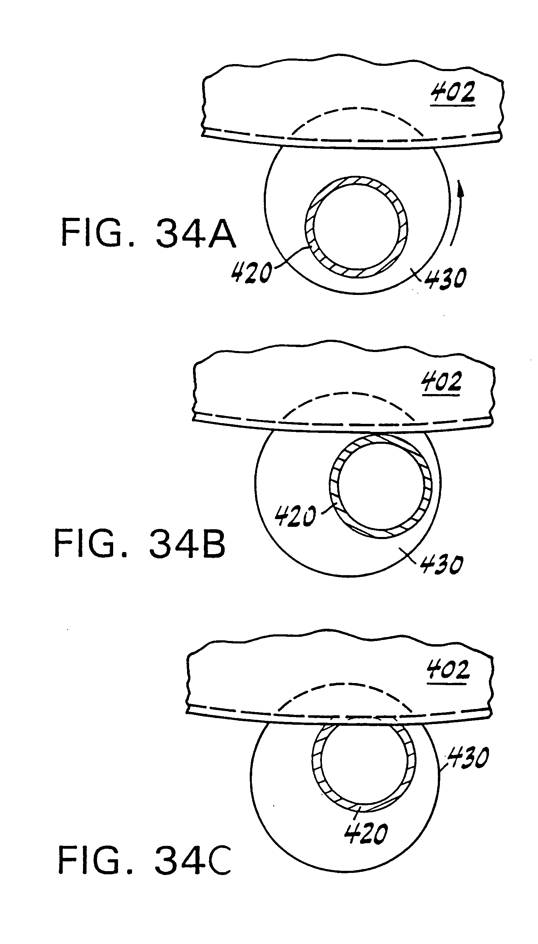

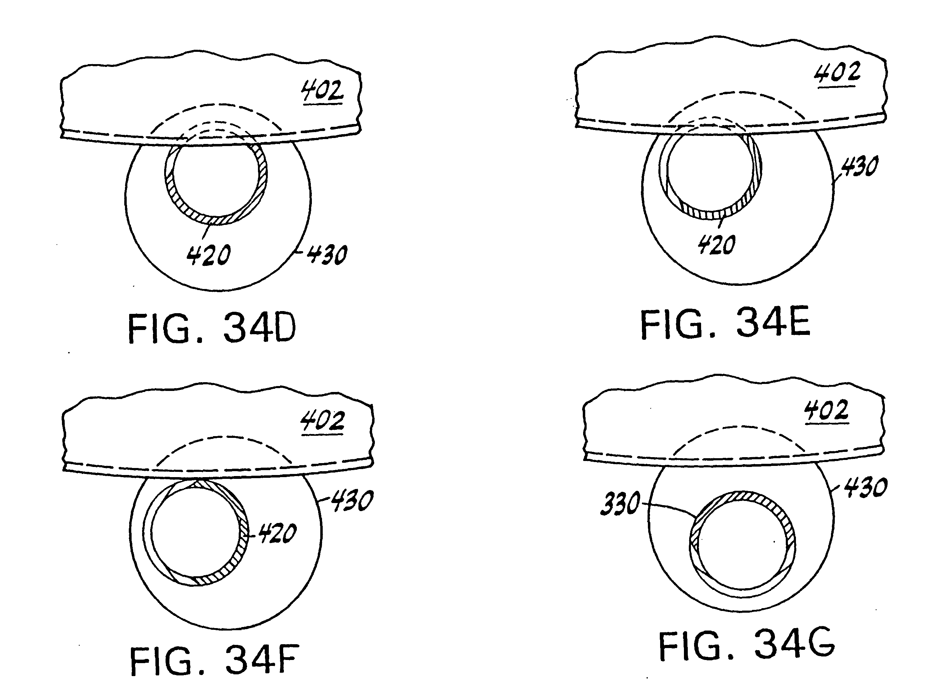

Jig 402 supports a rotatable housing 430 which is adapted to rotate about an axis 432 parallel to wheel axis 414. Extending from the front face 434 of housing 430 is a tubular workpiece 420 which has a distal tip 436 and an axis 438 which is aligned parallel to and offset by a distance D from axis 432 as best seen in Figures 28 and 30. The distance 439 between axis 432 and the radially innermost portion 417 is less than the sum of the radius T of the tubular member and the distance D between the tubular member axis 438 and housing axis 432. Wheel 404 is adapted to rotate about fixed axis 414 while the workpiece 420 is adapted to move in an eccentric pattern about axis 432. As shown in Figures 29-33, the workpiece does not rotate about its axis 438 but rotation of housing 430 about axis 432 causes the workpiece axis 438 to be placed at variable radial distances from the wheel axis 414 such that different parts of the workpiece 420 will intersect the perimetral surface 406 of the wheel at different points. The eccentric motion of workpiece 420 about axis 432, as best seen in Figure 33, causes points at varying radial distances from axis 432 to rotate along different circular paths. Thus, the circular path 441 is that defined by points on workpiece 420 that rotate about axis 432 at a radial distance from axis 432 equal to the distance between axis 432 and innermost portion 417 of the wheel's perimetral surface. Similarly, the circular path 442 is defined by points on workpiece 420 that rotate about axis 432 at a radial distance from axis 432 equal to the distance between axis 432 and outermost portion 416. It will be understood that various parts of the workpiece rotate along curvi-linear paths, in this case circular paths having radii between paths 441 and 442. As shown in Figure 34a-g the rotation of workpiece 420 relative to wheel 402 proceeds in various stages until ultimately, in Figure 34g, an outer tubular member 330 is produced.

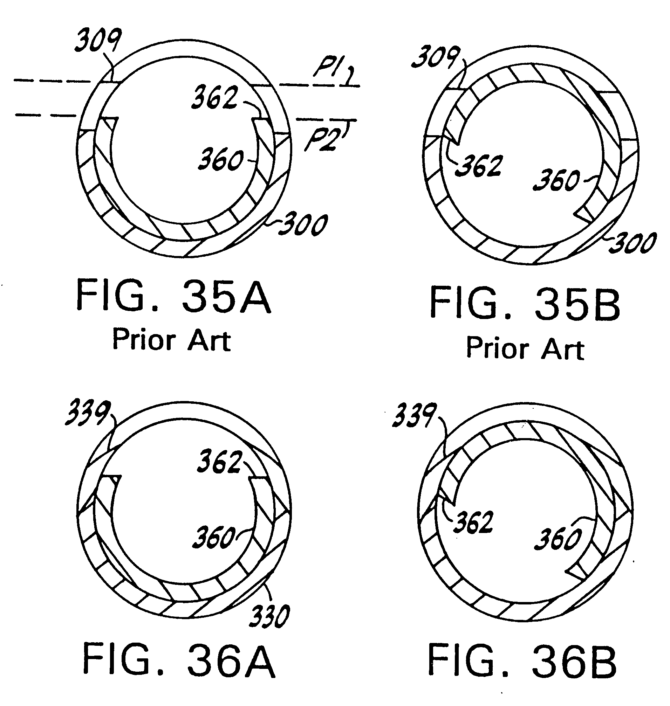

The sharper teeth of an outer tubular member that can be easily produced with this method produce a sharper rotatable surgical shaver. As shown in Figure 35, a prior art surgical shaver comprising an outer tubular member 300 and inner tubular member 360 is limited in sharpness. Outer tubular member 300 is identical to that shown in Figures 22-24. Inner tubular member 360 is shown also with a straight cut through its cutting window so that all of the cutting surfaces lie in parallel planes P1 and P2 (parallel to the plane of the inner window), as best seen in Figure 35a. (Note that a sharper inner member 50 is shown in Figures 8-11 with cutting surfaces 52 and 54 in parallel, longitudinal planes which are perpendicular to the inner window. Member 50 could be combined with outer member 300.) When inner member 360 is rotated relative to the outer member 300, the movement of cutting surface 362 past cutting edge 309 creates a shearing action. The actual cut occurs at the junction of the radially innermost portion of edge 309 and the radially outermost edge of surface 362. Because of the straight cuts used to form the cutting windows of such prior art devices, the included angle of these edges is large and, therefore, their sharpness is limited.

On the contrary, as shown in Figure 36, simply replacing a conventional outer member 300 with an outer tubular member 330 constructed in accordance with the principles of this invention will increase the sharpness of the junction between the radially innermost portion of edge 339 and the radially outermost edge of surface 362 thereby producing a smaller included angle and a sharper cut. It will be understood that inner member 360 may be produced with a toothed or untoothed cutting window. In either case, the cross-section of the inner member shown in Figure 35 and 36 will be similar.

The sharpened, non-toothed inner member shown in Figures 8-11 as inner member 50 could also be combined with outer member 330 to produce a sharper cutting action than that offered by the combination of Figure 35. If the inner member is additionally provided with teeth that are sharpened to a point at the radially outermost portion of each tooth (adjacent the innermost portion of edges 309 or 339) the sharpness of the cut will be even further enhanced.

One method of forming such a sharpened toothed or non-toothed inner member is shown in Figures 37 and 38 in which an electrochemical grinding machine or an electrical discharge machine may be used to produce sharpened edges or teeth in the cutting window of an inner tubular member 500. Tool 502 could be a rotating wheel in the case of an electrochemical grinding machine or a non-rotating electrode in the case of an electrical discharge machine. In each case the tool has a diameter smaller than that of the tubular member so the sharpened edge is formed on the radially outermost surface of the member. Moving the tool toward inner member 500 such that the intersection of tool 502 with the outer surface of tubular member 500 would produce opposing sharpened outer edges 504 and 506. The edges could be produced with or without teeth. It will be understood that replacing member 360 of Figure 36 with inner tubular member 500 will replace flat surface 362 with an angled surface 508 thereby producing a sharper cut.

It will be understood by those skilled in the art that numerous improvements and modifications may be made to the preferred embodiment of the invention disclosed herein without departing from the scope thereof.

## Claims

1. Method of forming an opening (34) at one end (32) of an elongated tubular surgical shaver blade (30, 220) having a distal end (32) and a proximal end, characterised by the steps of:
providing an apparatus (200) for performing an electro-chemical grinding process on a portion of the elongated tubular surgical shaver blade, the apparatus having a rotatable abrasive wheel (202) with a perimetral surface (230) which has a circumferential groove (232) formed therein, said groove having a predetermined arcuate profile in a radial plane of said wheel;
orienting the distal end of the elongated tubular surgical shaver blade (220) in a predetermined orientation relative to said wheel (202);
activating said apparatus (200) to perform an electro-chemical grinding process; and
moving the distal end of the elongated tubular shaver blade (220) relative to said wheel (202) during the performance of said electrochemical grinding process to perform the electrochemical grinding process on the distal end of the elongated tubular surgical shaver blade to thereby form an opening therein, where the opening (34) has a peripheral land (38) angled around the entire periphery (36) of the opening and where the periphery (36) has a sharp edge without necessity for a subsequent sharpening operation.

2. A method according to claim 1 wherein said wheel is planar, wherein said elongated tubular surgical shaver blade has an axis and wherein said step of orienting further comprises securing the tubular shaver blade in a fixed position with its axis aligned within the plane of said wheel and further comprising the step of moving the distal end of the elongated tubular surgical shaver blade tangentially relative to said wheel.

3. A method according to claim 1 wherein said predetermined arcuate profile comprises an arc having a predetermined radius of curvature.

4. A method according to claim 1 wherein said distal end of the elongated tubular surgical shaver blade comprises an end which is initially closed.

5. A method according to claim 1 wherein said step of moving further comprises moving said elongated tubular shaver blade in a linear path relative to a predetermined tangential point on said wheel.

6. A method according to claim 1 wherein said step of moving further comprises moving said elongated tubular shaver blade in a curvilinear path relative to a predetermined tangential point on said wheel.

7. A method according to claim 6 wherein said predetermined orientation is such that said elongated tubular shaver blade is aligned within said radial plane of said wheel.

8. A method of shaping an opening (34) in a predetermined portion of a tubular member (220) of a rotary surgical shaver blade, said predetermined portion having an axis, said method being characterised by the steps of:
providing a hollow tubular member (220) having an axis, a distal end and a proximal end;
providing a grinding wheel (202) having an axis and a predetermined arcuate groove (232) in its perimetral surface (230), said groove for creating in said tubular member an arcuate surface having at least one predetermined radius of curvature;
orienting said axis of said predetermined portion of said tubular member at a predetermined angle relative to a line tangent to said wheel;
rotating said grinding wheel (202) about its axis;
advancing said predetermined portion of said tubular member along a path which intersects with a selected portion of said grinding wheel whereby such motion will result in an opening being shaped in said predetermined portion of said tubular member, where the opening (34) has a peripheral land (38) angled around the entire periphery (36) of the opening and where the periphery (36) has a sharp edge without necessity for a subsequent sharpening operation.

9. A method according to claim 8 wherein said predetermined portion of said tubular member is the distal tip thereof.

10. A method according to claim 8 wherein said grinding wheel is an electrode component of a electrochemical grinding apparatus.

11. A method according to claim 8 wherein said orienting step further comprises aligning the axis of said tubular member perpendicular to the axis of said wheel.

12. A method according to claim 8 wherein said orienting step further comprising aligning the axis of said tubular member parallel to the axis of said wheel.

13. A method of shaping an opening (34, 334) in a tubular member (330) of a rotary surgical shaver blade characterised by the steps of:
providing a cylindrical forming apparatus (202, 400) having a circumferential surface (230, 406) for shaping in said tubular member an opening, said circumferential surface situated at a predetermined radius from a first axis and being movable about said first axis;
fixedly mounting a hollow tubular member (330) with its axis parallel to said first axis;
rotating said axis of said tubular member about a second axis parallel to said first axis, the distance between said first and second axes being greater than said predetermined radius and such that during rotation of said axis of said tubular member about said second axis a predetermined portion of said tubular member will intersect a predetermined portion of said circumferential surface, such that an opening (34) will be shaped where the opening (34) has a peripheral land (38) angled around the entire periphery (36) of the opening and where the periphery (36) has sharp edge without necessity for a subsequent sharpening operation.

14. A method according to claim 13 wherein said tubular member is fixedly mounted with its distal end adjacent said circumferential surface.

15. A method according to claim 13 wherein said tubular member has a closed distal end.

16. A method according to claim 13 wherein said circumferential surface (406) further comprises a predetermined profile having a plurality of circumferential notches (408) adapted to produce a toothed opening in said tubular member.

17. A method according to claim 13 wherein said circumferential surface comprises, relative to said first axis, a radially outermost portion and a radially innermost portion, wherein said circumferential surface rotates about said first axis and said tubular member rotates about said second axis, said second axis being spaced from said radially innermost portion by a distance which is less than the sum of the radius of said tubular member and the distance between said tubular axis and said second axis.

18. A method of forming a window (34) in a rotary surgical shaver blade characterised by the steps of:
providing a hollow tubular member (110) having a distal end (112) ;
providing an elongated arcuate surface forming means (106) comprising a first elongated arcuate concave surface (134) which is linear in the direction of elongation and which has a predetermined transverse curvilinear shape for, upon operation thereof, creating in said hollow tubular member (110) at least one second arcuate convex surface complementary to a predetermined portion of said first arcuate surface;
orienting said first arcuate concave surface (134) at a predetermined orientation relative to and facing said hollow tubular member;
advancing said first arcuate concave surface (134) toward said end (112) and operating said first arcuate concave surface to cause it to remove a predetermined portion of said hollow tubular member to create in said hollow tubular member a window (34) defined by the intersection of said hollow tubular member and said first arcuate surface, where the window (34) has a peripheral land (38) angled around the entire periphery (36) of the window and where the periphery (36) has a sharp edge without necessity for a subsequent sharpening operation.

19. A method according to claim 18 wherein said elongated arcuate surface forming means (106) is an electrode component of an electrical forming device (100) and wherein said first elongated concave arcuate surface (134) comprises an activatable electrode surface thereof.

20. A method according to claim 18 wherein said first arcuate surface (134) is elongated in a first direction and wherein a transverse cross-section of said first arcuate surface in a plane perpendicular to said first direction comprises, at all operative points along the length of said first arcuate surface, a curve having a predetermined radius of curvature (R3).

21. A method according to claim 18 wherein said transverse cross-section is the same at all points along the length of said first arcuate surface.

22. A method according to claim 18 wherein said distal end of said hollow tubular member has an axis and wherein said step of orienting further comprises:
positioning said first arcuate surface (134) at a predetermined angle (C) relative to said axis of said distal end of said hollow tubular member such that at least one longitudinally extending imaginary line, parallel to said axis and lying on said first arcuate surface will, upon formation of the window, intersect said axis at a predetermined point distal to said distal end of said hollow tubular member.

23. A method according to claim 22 wherein said first arcuate surface (134) is symmetrical about a line of symmetry and wherein said line of symmetry intersects said axis at a predetermined point distal to said distal end of said hollow tubular member.

## Patentansprüche

1. Verfahren zur Bildung einer Öffnung (34) an einem Ende (32) eines länglichen rohrförmigen chirurgischen Schabmessers (30, 220) mit einem distalen Ende (32) und einem proximalen Ende, gekennzeichnet durch die Schritte:
Bereitstellen einer Vorrichtung (200) zum Durchführen eines elektrochemischen Schleifverfahrens an einem Abschnitt des länglichen rohrförmigen chirurgischen Schabmessers, wobei die Vorrichtung eine drehbare Schleifscheibe (202) mit einer Begrenzungsfläche (230) hat, in der eine Umfangsnut (232) gebildet ist, wobei die Nut ein vorbestimmtes Bogenprofil in einer Radialebene der Scheibe hat;
Orientieren des distalen Endes des länglichen rohrförmigen chirurgischen Schabmessers (220) in einer vorbestimmten Orientierung relativ zur Scheibe (202) ;
Aktivieren der Vorrichtung (200), um ein elektrochemisches Schleifverfahren durchzuführen; und
Bewegen des distalen Endes des länglichen rohrförmigen chirurgischen Schabmessers (220) relativ zur Scheibe (202) während der Durchführung des elektrochemischen Schleifverfahrens am distalen Ende des länglichen rohrförmigen chirurgischen Schabmessers, um dadurch eine Öffnung darin zu bilden, wobei die Öffnung (34) eine um die gesamte Peripherie (36) der Öffnung abgewinkelte periphere Fase (38) hat und wobei die Peripherie (36) eine scharfe Kante hat, ohne daß ein anschließender Schärfvorgang notwendig ist.

2. Verfahren nach Anspruch 1, wobei die Scheibe eben ist, wobei das längliche rohrförmige chirurgische Schabmesser eine Achse hat und wobei der Schritt des Orientierens ferner den Schritt aufweist: Befestigen des rohrförmigen Schabmessers in einer festen Position, wobei seine Achse innerhalb der Ebene der Scheibe ausgerichtet ist, und ferner mit dem Schritt: tangentiales Bewegen des distalen Endes des länglichen rohrförmigen chirurgischen Schabmessers relativ zur Scheibe.

3. Verfahren nach Anspruch 1, wobei das vorbestimmte Bogenprofil einen Bogen mit einem vorbestimmten Krümmungsradius aufweist.

4. Verfahren nach Anspruch 1, wobei das distale Ende des länglichen rohrförmigen chirurgischen Schabmessers ein Ende aufweist, das anfänglich geschlossen ist.

5. Verfahren nach Anspruch 1, wobei der Schritt des Bewegens ferner den Schritt aufweist: Bewegen des länglichen rohrförmigen Schabmessers auf einem geradlinigen Weg relativ zu einem vorbestimmten Tangentialpunkt an der Scheibe.

6. Verfahren nach Anspruch 1, wobei der Schritt des Bewegens ferner den Schritt aufweist: Bewegen des länglichen rohrförmigen Schabmessers auf einem krummlinigen Weg relativ zu einem vorbestimmten Tangentialpunkt an der Scheibe.

7. Verfahren nach Anspruch 6, wobei die vorbestimmte Orientierung so ist, daß das längliche rohrförmige Schabmesser innerhalb der Radialebene der Scheibe ausgerichtet ist.

8. Verfahren zum Formen einer Öffnung (34) in einem vorbestimmten Abschnitt eines Rohrteils (220) eines chirurgischen Drehschabmessers, wobei der vorbestimmte Abschnitt eine Achse hat, wobei das Verfahren durch die Schritte gekennzeichnet ist:
Bereitstellen eines hohlen Rohrteils (220) mit einer Achse, einem distalen Ende und einem proximalen Ende;
Bereitstellen einer Schleifscheibe (202) mit einer Achse und einer vorbestimmten Bogennut (232) in ihrer Begrenzungsfläche (230), wobei die Nut zum Erzeugen einer gebogenen Oberfläche mit mindestens einem vorbestimmten Krümmungsradius im Rohrteil dient;
Orientieren der Achse des vorbestimmten Abschnitts des Rohrteils in einem vorbestimmten Winkel relativ zu einer Tangente zur Scheibe;
Drehen der Schleifscheibe (202) um ihre Achse;
Vorbewegen des vorbestimmten Abschnitts des Rohrteils auf einem Weg, der einen ausgewählten Abschnitt der Schleifscheibe schneidet, wodurch eine solche Bewegung dazu führt, daß eine Öffnung im vorbestimmten Abschnitt des Rohrteils geformt wird, wobei die Öffnung eine um die gesamte Peripherie (36) der Öffnung abgewinkelte periphere Fase (38) hat und wobei die Peripherie (36) eine scharfe Kante hat, ohne daß ein anschließender Schärfvorgang notwendig ist.

9. Verfahren nach Anspruch 8, wobei der vorbestimmte Abschnitt des Rohrteils dessen distale Spitze ist.

10. Verfahren nach Anspruch 8, wobei die Schleifscheibe eine Elektrodenkomponente einer elektrochemischen Schleifvorrichtung ist.

11. Verfahren nach Anspruch 8, wobei der Orientierungsschritt ferner den folgenden Schritt aufweist: lotrechtes Ausrichten der Achse des Rohrteils zur Achse der Scheibe.

12. Verfahren nach Anspruch 8, wobei der Orientierungsschritt ferner den Schritt aufweist: paralleles Ausrichten der Achse des Rohrteils zur Achse der Scheibe.

13. Verfahren zum Formen einer Öffnung (34, 334) in einem Rohrteil (330) eines chirurgischen Drehschabmessers, gekennzeichnet durch die Schritte:
Bereitstellen einer zylindrischen Formgebungsvorrichtung (202, 400) mit einer Umfangsfläche (230, 406) zum Formen einer Öffnung im Rohrteil, wobei sich die Umfangsfläche in einem vorbestimmten Radius von einer ersten Achse befindet und um die erste Achse beweglich ist;
festes Anordnen eines hohlen Rohrteils (330), wobei seine Achse parallel zur ersten Achse ist;
Drehen der Achse des Rohrteils um eine zweite Achse parallel zur ersten Achse, wobei der Abstand zwischen der ersten und zweiten Achse größer als der vorbestimmte Radius ist, und so, daß während der Drehung der Achse des Rohrteils um die zweite Achse ein vorbestimmter Abschnitt des Rohrteils einen vorbestimmten Abschnitt der Umfangsfläche so schneidet, daß eine Öffnung (34) geformt wird, wobei die Öffnung (34) eine um die gesamte Peripherie (36) der Öffnung abgewinkelte periphere Fase (38) hat und wobei die Peripherie (36) eine scharfe Kante hat, ohne daß ein anschließender Schärfvorgang notwendig ist.

14. Verfahren nach Anspruch 13, wobei das Rohrteil mit seinem distalen Ende benachbart zur Umfangsfläche fest angeordnet ist.

15. Verfahren nach Anspruch 13, wobei das Rohrteil ein geschlossenes distales Ende hat.

16. Verfahren nach Anspruch 13, wobei die Umfangsfläche (406) ferner ein vorbestimmtes Profil mit mehreren Umfangskerben (408) aufweist, die geeignet sind, eine gezahnte Öffnung im Rohrteil herzustellen.

17. Verfahren nach Anspruch 13, wobei die Umfangsfläche relativ zur ersten Achse einen radial am weitesten außenliegenden Abschnitt und einen radial am weitesten innenliegenden Abschnitt aufweist, wobei die Umfangsfläche um die erste Achse dreht und das Rohrteil um die zweite Achse dreht, wobei die zweite Achse vom radial am weitesten innenliegenden Abschnitt um einen Abstand beabstandet ist, der kleiner als die Summe aus dem Radius des Rohrteils und dem Abstand zwischen der Rohrteilachse und der zweiten Achse ist.

18. Verfahren zur Bildung eines Fensters (34) in einem chirurgischen Drehschabmesser, gekennzeichnet durch die Schritte:
Bereitstellen eines hohlen Rohrteils (110) mit einem distalen Ende (112);
Bereitstellen einer Einrichtung (106) zur Bildung einer länglichen gebogenen Oberfläche mit einer ersten länglichen gebogenen konkaven Oberfläche (134), die geradlinig in Elongationsrichtung ist und die eine vorbestimmte krummlinige Querform zum bei ihrer Einwirkung erfolgenden Erzeugen mindestens einer zweiten gebogenen konvexen Oberfläche im hohlen Rohrteil (110) hat, die komplementär zu einem vorbestimmten Abschnitt der ersten gebogenen Oberfläche ist;
Orientieren der ersten gebogenen konkaven Oberfläche (134) in einer vorbestimmten Orientierung relativ zum hohlen Rohrteil und zu ihm weisend;
Vorbewegen der ersten gebogenen konkaven Oberfläche (134) zum Ende (112) und Einwirkenlassen der ersten gebogenen konkaven Oberfläche, damit sie einen vorbestimmten Abschnitt des hohlen Rohrteils entfernt und im hohlen Rohrteil ein Fenster (34) erzeugt, das durch den Schnitt des hohlen Rohrteils und der ersten gebogenen Oberfläche gebildet ist, wobei das Fenster (34) eine um die gesamte Peripherie (36) des Fensters abgewinkelte periphere Fase (38) hat und wobei die Peripherie (36) eine scharfe Kante hat, ohne daß ein anschließender Schärfvorgang notwendig ist.

19. Verfahren nach Anspruch 18, wobei die Einrichtung (106) zur Bildung einer länglichen gebogenen Oberfläche eine Elektrodenkomponente einer elektrischen Formgebungsvorrichtung (100) ist und wobei die erste längliche konkave gebogene Oberfläche (134) eine aktivierbare Elektrodenfläche aufweist.

20. Verfahren nach Anspruch 18, wobei die erste gebogene Oberfläche (134) in einer ersten Richtung länglich ist und wobei ein quer verlaufender Querschnitt der ersten gebogenen Oberfläche in einer Ebene lotrecht zur ersten Richtung an allen Arbeitspunkten über die Länge der ersten gebogenen Oberfläche eine Krümmung mit einem vorbestimmten Krümmungsradius (R3) aufweist.

21. Verfahren nach Anspruch 18, wobei der quer verlaufende Querschnitt an allen Punkten über die Länge der ersten gebogenen Oberfläche gleich ist.

22. Verfahren nach Anspruch 18, wobei das distale Ende des hohlen Rohrteils eine Achse hat und wobei der Schritt des Orientierens ferner den Schritt aufweist:
Positionieren der ersten gebogenen Oberfläche (134) in einem vorbestimmten Winkel (C) relativ zur Achse des distalen Endes des hohlen Rohrteils, so daß mindestens eine sich längs erstreckende gedachte Linie, die parallel zur Achse ist und auf der ersten gebogenen Oberfläche liegt, bei Bildung des Fensters die Achse an einem vorbestimmten Punkt distal zum distalen Ende des hohlen Rohrteils schneidet.

23. Verfahren nach Anspruch 22, wobei die erste gebogene Oberfläche (134) symmetrisch zu einer Symmetrielinie ist und wobei die Symmetrielinie die Achse an einem vorbestimmten Punkt distal zum distalen Ende des hohlen Rohrteils schneidet.

## Revendications

1. Procédé pour réaliser une ouverture (34) à une extrémité (32) d'un appareil de résection chirurgical tubulaire allongé (30, 220) ayant une extrémité distale (32) et une extrémité proximale, caractérisé par les étapes consistant à :
prévoir un appareil (200) pour exécuter un processus de meulage électrochimique sur une portion de l'appareil de résection chirurgical tubulaire allongé, l'appareil comportant une meule abrasive tournante (202) avec une surface périmétrique (230) qui présente une rainure circonférentielle (232) ménagée dans celle-ci, ladite rainure ayant un profil arqué prédéterminé dans un plan radial de ladite meule ;
orienter l'extrémité distale de l'appareil de résection chirurgical tubulaire allongé (220) dans une orientation prédéterminée relativement à ladite meule (202) ;
activer ledit appareil (200) pour exécuter un processus de meulage électrochimique ; et
déplacer l'extrémité distale de l'appareil de résection tubulaire allongé (220) relativement à ladite meule (202) pendant l'exécution dudit processus de meulage électrochimique pour effectuer le processus de meulage électrochimique sur l'extrémité distale de l'appareil de résection chirurgical tubulaire allongé pour ménager ainsi une ouverture dans celle-ci, où l'ouverture (34) a une zone périphérique (38) pliée autour de toute la périphérie (36) de l'ouverture, et où la périphérie (36) a un bord tranchant sans qu'une opération d'affûtage ultérieure soit nécessaire.

2. Procédé selon la revendication 1, où ladite meule est plane, où ledit appareil de résection chirurgical tubulaire allongé a un axe et où ladite étape d'orientation comprend en outre la fixation de l'appareil de résection tubulaire dans une position fixe où son axe est aligné avec le plan de ladite meule, et comprenant en outre l'étape consistant à déplacer l'extrémité distale dudit appareil de résection chirurgical tubulaire allongé tangentiellement relativement à ladite meule.

3. Procédé selon la revendication 1, où ledit profil arqué prédéterminé comprend un arc ayant un rayon de courbure prédéterminé.

4. Procédé selon la revendication 1, où ladite extrémité distale de l'appareil de résection chirurgical tubulaire allongé comprend une extrémité qui est initialement fermée.

5. Procédé selon la revendication 1, où ladite étape de déplacement comprend en outre le déplacement dudit appareil de résection chirurgical tubulaire allongé selon un chemin linéaire relativement à un point tangentiel prédéterminé sur ladite meule.

6. Procédé selon la revendication 1, où ladite étape de déplacement comprend en outre le déplacement dudit appareil de résection tubulaire allongé selon un chemin curviligne relativement à un point tangentiel prédéterminé sur ladite meule.

7. Prccédé selon la revendication 6, où ladite orientation prédéterminée est telle que ledit appareil de résection tubulaire allongé est aligné avec ledit plan radial de ladite meule.

8. Procédé pour configurer une ouverture (34) dans une portion prédéterminée d'un élément tubulaire (220) d'un appareil de résection chirurgical rotatif, ladite portion prédéterminée ayant un axe, ledit procédé étant caractérisé par les étapes consistant à :
prévoir un élément tubulaire creux (220) ayant un axe, une extrémité distale et une extrémité proximale ;
prévoir une meule (202) ayant un axe et une rainure arquée prédéterminée (232) dans sa surface périmétrique (230), ladite rainure pour créer dans ledit élément tubulaire une surface arquée ayant au moins un rayon de courbure prédéterminé ;
orienter ledit axe de ladite portion prédéterminée dudit élément tubulaire selon un angle prédéterminé relativement à une ligne tangente à ladite meule ;
faire tourner ladite meule (202) autour de son axe ;
faire avancer ladite portion prédéterminée dudit élément tubulaire le long d'un chemin qui se croise avec une portion sélectionnée de ladite meule par quoi un tel mouvement se traduira par la formation d'une ouverture dans ladite portion prédéterminée dudit élément tubulaire, où l'ouverture (34) présente une zone périphérique (38) pliée autour de toute la périphérie (36) de l'ouverture, et où la périphérie (36) a un bord tranchant sans qu'une opération d'affûtage ultérieure soit nécessaire.

9. Procédé selon la revendication 8, où ladite portion prédéterminée dudit élément tubulaire est la pointe distale de celui-ci.

10. Procédé selon la revendication B, où la meule est un composant d'électrode d'un appareil de meulage électrochimique.

11. Procédé selon la revendication 8, où ladite étape d'orientation comprend en outre l'alignement de l'axe dudit élément tubulaire perpendiculairement à l'axe de ladite meule.

12. Procédé selon la revendication 8, où ladite étape d'orientation comprend en outre l'alignement de l'axe dudit élément tubulaire parallèlement à l'axe de ladite roue.

13. Procédé de configuration d'une ouverture (34, 334) dans un élément tubulaire (330) d'un appareil de résection chirurgical rotatif, caractérisé par les étapes consistant à :
prévoir un appareil de formage cylindrique (202, 400) ayant une surface circonférentielle (230, 406) pour ménager dans ledit élément tubulaire une ouverture, ladite surface circonférentielle étant située à un rayon prédéterminé d'un premier axe et étant déplaçable autour dudit premier axe ;
monter fixement un élément tubulaire creux (330) avec son axe parallèle audit premier axe ;
faire tourner ledit axe dudit élément tubulaire autour d'un second axe parallèle audit premier axe, la distance entre lesdits premier et second axes étant plus grande que ledit rayon prédéterminé et de façon que pendant la rotation dudit axe dudit élément tubulaire autour dudit second axe, une portion prédéterminé dudit élément tubulaire se croisera avec une portion prédéterminée de ladite surface circonférentielle de façon qu'une ouverture (34) sera formée, où l'ouverture (34) a une zone périphérique (38) pliée autour de toute la périphérie (36) de l'ouverture, et où la périphérie (36) a un bord tranchant sans qu'une opération d'affûtage ultérieure soit nécessaire.

14. Procédé selon la revendication 13, où ledit élément tubulaire est monté fixement avec son extrémité distale adjacente à ladite surface circonférentielle.

15. Procédé selon la revendication 13, où ledit élément tubulaire a une extrémité distale fermée.

16. Procédé selon la revendication 13, où ladite surface circonférentielle (406) comprend en outre un profil prédéterminé présentant plusieurs encoches circonférentielles (408) aptes à produire une ouverture dentée dans ledit élément tubulaire.

17. Procédé selon la revendication 13, où ladite surface circonférentielle comprend, relativement audit premier axe, une portion radialement la plus externe et une portion radialement la plus interne, où ladite surface circonférentielle tourne autour dudit premier axe, et ledit élément tubulaire tourne autour dudit second axe, ledit second axe étant espacé de ladite portion radialement la plus interne selon une distance qui est inférieure à la somme du rayon dudit élément tubulaire et de la distance entre ledit axe tubulaire et ledit second axe.

18. Procédé pour former une fenêtre (34) dans un appareil de résection chirurgical rotatif, caractérisé par les étapes consistant à :
prévoir un élément tubulaire creux (110) ayant une extrémité distale (112) ;
prévoir un moyen de formage de surface arquée allongée (106) comprenant une première surface concave arquée allongée (134) qui est linéaire dans la direction d'allongement et qui a une forme curviligne transversale prédéterminée pour, lors du fonctionnement de celle-ci, créer dans ledit élément tubulaire creux (110) au moins une seconde surface convexe arquée complémentaire à une portion prédéterminée de ladite première surface arquée ;
orienter ladite première surface concave arquée (134) selon une orientation prédéterminée relativement à et tournée vers ledit: élément tubulaire creux ;
faire avancer ladite première surface concave arquée (134) vers ladite extrémité (112) et faire fonctionner ladite première surface concave arquée pour l'amener à retirer une portion prédéterminée dudit élément tubulaire creux pour créer dans ledit élément tubulaire creux une fenêtre (34) définie par l'intersection dudit élément tubulaire creux et de ladite première surface arquée, où la fenêtre (34) a une zone périphérique (38) qui est pliée autour de toute la périphérie (38) de la fenêtre, et où la périphérie (36) a un bord tranchant sans qu'une opération d'affûtage ultérieure soit nécessaire.

19. Procédé selon la revendication 18, où ledit moyen de formage de surface arquée allongée (106) est un composant d'électrode d'un dispositif de formage électrique (100) et où ladite première surface arquée concave allongée (134) comprend une surface d'électrode apte à être activée de celui.

20. Procédé selon la revendication 18, où ladite première surface arquée (134) est allongée dans une première direction, et où une section transversale de ladite première surface arquée dans un plan perpendiculaire à ladite première direction comprend, à tous les points fonctionnels sur la longueur de ladite première surface arquée, une courbe d'un rayon de courbure prédéterminée (R3).

21. Procédé selon la revendication 18, où ladite section transversale est la même à tous les points sur la longueur de ladite première surface arquée.

22. Procédé selon la revendication 18, où ladite extrémité distale dudit élément tubulaire creux a un axe, et où ladite étape d'orientation comprend en outre :
positionner ladite première surface arquée (134) selon un angle prédéterminé (C) relativement audit axe de ladite extrémité distale dudit élément tubulaire creux de façon qu'au moins une ligne imaginaire s'étendant longitudinalement, parallèle audit axe et se situant sur ladite première surface arquée, lors de la formation de la fenêtre, se croisera avec ledit axe à un point prédéterminé distal à ladite extrémite distale dudit élément tubulaire creux.

23. Procédé selon la revendication 22, où ladite première surface arquée (134) est symétrique autour dune ligne de symétrie, et où ladite ligne de symétrie croise ledit axe à un point prédéterminé distal à ladite extrémité distale dudit élément tubulaire creux.
